# EUROPÄISCHE PATENTANMELDUNG

(11) **EP 4 759 334 A1**
(43) Veröffentlichungstag der Anmeldung: **17.06.2026**
(21) Anmeldenummer: 24221946.7
(22) Anmeldetag: 19.12.2024
(51) Int. Cl.: A61L 2/26, B01L 1/02, A61L 2/24, B25J 21/00, B25J 21/02, C12M 1/12, F24F 3/167, B65B 3/00, B65B 55/02

(54) **EINSCHLEUSEVORRICHTUNG FÜR EINE KONTROLLIERTE UMGEBUNG**

(30) Priorität: 16.12.2024 EP 24220378
(71) Anmelder: TT Innovation AG, 6343 Rotkreuz (CH)
(72) Erfinder: ZELLER, Mike, 4246 Wahlen (CH); MÜLLER, Mathieu, 68510 Sierentz (FR); LANDES, Robert, 79713 Bad Säckingen (DE)
(74) Vertreter: Mertzlufft-Paufler, Cornelius

(57) **Zusammenfassung**

Es wird ein Verfahren zum Einschleusen von Objekten (7) in eine kontrollierte Umgebung (3) und eine
Einschleusevorrichtung (1) mit einer eine Passage (2) aufweisenden kontrollierten Umgebung (3) vorgeschlagen, wobei die Passage (2) mit einem Deckel (5) von innen (4), insbesondere innerhalb der kontrollierten Umgebung (3), und mit einer ein einzuschleusendes Objekt (7) enthaltenden Verpackung (6) von außen (8) verschließbar ist, und mit einer Dekontaminationsvorrichtung (9), die zu einer Dekontamination der Passage (2) eingerichtet ist, wobei eine
Überprüfungseinrichtung (10) zu einer Überprüfung einer Funktion der Dekontaminationsvorrichtung (9) eingerichtet ist

## Beschreibung

Die Erfindung betrifft eine Einschleusevorrichtung mit einer eine Passage aufweisenden kontrollierten Umgebung, wobei die Passage mit einem Deckel von innen, insbesondere innerhalb der kontrollierten Umgebung, und einer ein einzuschleusendes Objekt enthaltenden Verpackung von außen verschließbar ist, und mit einer Dekontaminationsvorrichtung, die zu einer Dekontamination der Passage eingerichtet ist.

Die Erfindung betrifft weiter ein Verfahren zum Einschleusen eines Objektes in eine kontrollierte Umgebung, wobei die Verpackung einer Dekontamination ausgesetzt wird, bevor das Objekt in die kontrollierte Umgebung gebracht wird.

Aufgabe der Erfindung ist es derartige Einschleusevorrichtungen zu verbessern. Aufgabe der Erfindung ist es auch derartige Verfahren zu verbessern.

Zur Lösung der Aufgabe sind erfindungsgemäß die Merkmale des Anspruchs 1 vorgesehen. Insbesondere wird somit zur Lösung der genannten Aufgabe bei einer Einschleusevorrichtung der eingangs beschriebenen Art vorgeschlagen, dass eine Überprüfungseinrichtung zu einer Überprüfung zumindest einer Funktion der Dekontaminationsvorrichtung eingerichtet ist. Hierdurch kann beispielsweise erreichbar sein, dass ein Fehler oder Mangel der Dekontaminationsvorrichtung oder ein Abfall oder ein Ausfall der Funktion der Dekontaminationsvorrichtung vorzugsweise automatisch und/oder zeitnah erfassbar ist. Die Funktion der Dekontaminationsvorrichtung kann als Dekontaminationsfunktion definierbar sein. Die Einschleusevorrichtung kann zum Einschleusen von Objekten, beispielsweise medizinischen Behältern, insbesondere Vials, Spritzen, Karpulen (Zylinderampullen), in die kontrollierte Umgebung eingerichtet sein. Objekte können auch weitere Gegenstände umfassen wie beispielsweise Notfallhandschuhe, Pinzetten, Verbrauchsmaterial, Schläuche, Stopfen, Spritzen und/oder Beutel. Die Einschleusevorrichtung kann auch ein Rapid Transfer Port (RTP) sein. Die Verpackung des Objekts kann dabei die Passage, durch die die Objekte in die kontrollierte Umgebung eingeschleust werden können, von außen verschließen. Außen kann dabei beispielsweise als eine Umgebung außerhalb der kontrollierten Umgebung definiert sein. Außen kann dabei beispielsweise eine weniger kontrollierte Umgebung als die kontrollierte Umgebung sein. Außen kann aber beispielsweise auch eine zweite kontrollierte Umgebung sein. Der Deckel kann die Passage von innen, also von innerhalb der kontrollierten Umgebung verschließen. Die Verpackung kann ein sogenanntes Tub sein, das eine Behältnisaufnahme für medizinische und/oder pharmazeutische Behälter bildet. Die Verpackung kann dabei eine Abdeckung aus einem Vliesgewebe, wie beispielsweise Tyvek, aufweisen. Die Verpackung kann vor einem Zuführen an die Passage in einer Umverpackung gehalten sein. Diese Umverpackung kann vor der Dekontamination entfernt werden. In der Passage kann eine Dichtung sitzen, welche zusammen mit der Verpackung und/oder mit dem angepressten Deckel an der Passage eine Abdichtung schafft, so dass kein Luftaustausch zwischen der kontrollierten Umgebung und außerhalb der kontrollierten Umgebung stattfinden kann. Die Verpackung kann von außen mit einer Handlingsvorrichtung an die Passage angepresst werden, wobei so mit der Verpackung und der kontrollierten Umgebung eine Abdichtung erreicht werden kann. Alternativ kann die Verpackung durch einen Klemmmechanismus, manuell oder automatisiert klemmend, an die Passage, insbesondere die darin befindliche Dichtung angelegt werden. Eine Abdichtung kann beispielsweise auch dadurch erreichbar sein, dass die Verpackung von außerhalb der kontrollierten Umgebung so an der Passage gehalten ist, dass ein Spalt zwischen der Verpackung und der kontrollierten Umgebung vorhanden ist. Durch diesen Spalt kann Luft von innerhalb der kontrollierten Umgebung nach außerhalb der kontrollierten Umgebung fließen, so dass Verunreinigungen nicht in die kontrollierte Umgebung eindringen können. Für eine Abdichtung muss also kein direkter Kontakt zwischen der Verpackung und der kontrollierten Umgebung vorhanden sein.

Die kontrollierte Umgebung kann beispielsweise als ein begrenzter Raumbereich charakterisierbar sein, in welchem definierte Umgebungsbedingungen, insbesondere in Bezug auf eine Luftreinheit und/oder eine Oberflächenreinheit, schaffbar und/oder aufrechterhaltbar sind, beispielsweise durch eine Kontrolle eines Luftaustausches mit einer Außenwelt der kontrollierten Umgebung. Die Kontrolle des Luftaustausches kann beispielsweise durch einen völligen Ausschluss des Luftaustausches im Normalbetrieb (beispielsweise bis auf eine geschlossene Luftzirkulation) oder eine konsequente Vorgabe einer Richtung des Luftaustausches (in die kontrollierte Umgebung oder aus der kontrollierten Umgebung) realisiert sein. Beispiele kontrollierter Umgebungen umfassen Isolatoren, Restricted Access Barrier Systems (zugangsbeschränkte Barrierensysteme, insbesondere vom offenen oder geschlossenen Typ), Containments, Gloveboxes.

Innerhalb der kontrollierten Umgebung, insbesondere des Containments oder des Isolators, können somit verschiedene Prozesse zur Verarbeitung verschiedenster Erzeugnisse, beispielsweise pharmazeutischer Erzeugnisse, erfolgen, wobei die Gefahr einer Kontamination wenigstens eines Erzeugnisses durch Partikel und/oder Verunreinigungen von außerhalb der kontrollierten Umgebung dezimiert werden kann. Solche pharmazeutischen Erzeugnisse können beispielsweise jeweils einen pharmazeutischen Behälter zu einer Aufnahme eines pharmazeutischen Präparats aufweisen. Eine Liste von Beispielen solcher pharmazeutischen Behälter umfasst zumindest Vials, Spritzen, Karpulen und sonstige befüllbare pharmazeutische Behälter.

Als die Passage kann beispielsweise der Rauminhalt, der durch die Passage und die angrenzenden Oberflächen, insbesondere Deckel und Verpackung, gebildet wird, verstanden werden.

Die kontrollierte Umgebung kann beispielsweise als ein Rauminhalt charakterisierbar sein, der bei verschlossener Passage eingeschlossen ist. Ein Öffnen der Passage würde bei diesem Verständnis nicht die kontrollierte Umgebung vergrößern, obwohl sich dann eingestellte Umgebungsbedingungen auf die Passage erstrecken würden. Vielmehr wäre in diesem Sinne durch ein Öffnen der Passage die kontrollierte Umgebung mit einem weiteren Raum zu einer Nivellierung von Umgebungsbedingungen verbunden.

Bei einer vorteilhaften Ausgestaltung kann vorgesehen sein, dass die Dekontaminationsvorrichtung eine Emissionseinheit aufweist. Hierdurch kann beispielsweise erreichbar sein, dass die Dekontaminationsvorrichtung Stoffe oder Strahlung emittieren kann, um damit ein oder mehrere Objekte zu dekontaminieren. Insbesondere kann die Dekontaminationsvorrichtung einen Strahlungsgenerator aufweisen. Hierdurch kann beispielsweise erreichbar sein, dass zu dekontaminierende Objekte mit Strahlung dekontaminiert werden können. Als Strahlung kann unter anderem Wärmestrahlung, energiereiche Teilchenstrahlung, wie e-Beam, Gammastrahlung und/oder Mikrowellenstrahlung zum Einsatz kommen. Ebenfalls kann ein Puls-Licht oder ein Plasma zur Dekontamination vorgesehen sein. Vorzugsweise weist der Strahlungsgenerator eine UVC-Lampe auf, womit Objekte dekontaminiert werden können. Alternativ oder zusätzlich kann die Dekontaminationsvorrichtung eine Dekontaminationsmittelverteilvorrichtung aufweisen mit der beispielsweise Stoffe verteilt werden können, um so Objekte zu dekontaminieren. Die Dekontaminationsmittelverteilvorrichtung kann beispielsweise zum Vernebeln, Versprühen, Einspritzen, Eindüsen oder Verdampfen eingerichtet sein. Insbesondere kann die Dekontaminationsmittelverteilvorrichtung ein H2O2-Vernebler sein. Hierdurch können beispielsweise Objekte mit H2O2 dekontaminiert werden. Ein Vernebeln kann als ein Versprühen und/oder Verdampfen von Stoffen definiert sein. Neben H2O2 (Wasserstoffperoxid) können auch andere, keimabtötende Chemikalien einsetzbar sein.

Bei einer vorteilhaften Ausgestaltung kann vorgesehen sein, dass die Dekontaminationsvorrichtung in dem Deckel angeordnet ist. Hierdurch kann beispielsweise der Deckel, der die Dekontaminationsvorrichtung umfassen kann, die Passage verschließen und währenddessen die Dekontamination von der in der Passage befindlichen Verpackung durchführen. Die Dekontaminationsvorrichtung kann dabei in den Deckel integriert sein. Alternativ oder zusätzlich kann die Dekontaminationsvorrichtung in der Passage angeordnet sein. Hierdurch kann beispielsweise erreichbar sein, dass die Passage und/oder die in der Passage befindliche Verpackung von der in der Passage angeordneten Dekontaminationsvorrichtung dekontaminiert werden kann. Zusätzlich vorteilhaft kann sein, dass die Passage und/oder die Verpackung von beiden Dekontaminationsvorrichtungen, der in der Passage und der in dem Deckel, dekontaminiert werden können. Dies kann die Wirksamkeit und Schnelligkeit der Dekontamination erhöhen. Die Dekontaminationsvorrichtung kann in der Passage integriert sein.

Zeitlich nach der Dekontamination kann die Einschleusung der in der Verpackung gehaltenen Behälter in die kontrollierte Umgebung erfolgen. Die eingeschleusten Behälter oder andere eingeschleuste Objekte können in der kontrollierten Umgebung an Prozessstationen, beispielsweise Befüllstationen oder Verschließstationen, weiterverarbeitet werden.

Bei einer vorteilhaften Ausgestaltung kann vorgesehen sein, dass die Überprüfungseinrichtung innerhalb der Passage angeordnet ist. Hierdurch kann die Überprüfungseinrichtung räumlich nahe zur Dekontamination angeordnet sein und beispielsweise die Funktion der Dekontaminationsvorrichtung dort, nämlich in der Passage, überprüfen, wo die Dekontamination stattfindet. Zusätzlich kann diese Anordnung platzsparend und effizient sein. Alternativ oder zusätzlich kann vorgesehen sein, dass die Überprüfungseinrichtung außerhalb der Passage angeordnet ist, beispielsweise in den Deckel integriert ist. Hierdurch kann beispielsweise die Passage, wenn die Überprüfungseinrichtung außerhalb der Passage angeordnet ist, weniger komplex aufgebaut sein.

Bei einer vorteilhaften Ausgestaltung kann vorgesehen sein, dass die Überprüfungseinrichtung wenigstens einen Sensor aufweist. Der wenigstens eine Sensor kann beispielsweise für die Messung von Prozessparametern eingerichtet sein, mit denen eine Funktion der Dekontaminationsvorrichtung ermittelt werden kann. Insbesondere kann der wenigstens eine Sensor in der Dekontaminationsvorrichtung angeordnet sein. Hierdurch kann beispielsweise erreichbar sein, dass mit dem Sensor die Funktion der Dekontaminationsvorrichtung während oder unmittelbar nach der Dekontamination überprüfbar ist. Ebenfalls kann vorteilhaft erreichbar sein, dass kein zusätzlicher Aufstellort für einen externen Sensor nötig ist. Ein Verfahren der Dekontaminationsvorrichtung zu einem externen Sensor kann entbehrlich sein. Insbesondere kann der wenigstens eine Sensor in der Dekontaminationsvorrichtung integriert sein. Alternativ oder zusätzlich kann der wenigstens eine Sensor außerhalb der Dekontaminationsvorrichtung angeordnet sein. Eine derartige Anordnung kann bauliche Vorteile mit sich bringen, da beispielsweise mehr Raum für die Emissionseinheit in der Dekontaminationsvorrichtung zur Verfügung stehen kann.

Ebenfalls kann dadurch vorteilhaft sein, dass die Dekontamination nicht behindert wird. Auch kann die Dekontamination durch den außerhalb der Dekontaminationsvorrichtung angeordneten Sensor vollständig erfassbar sein. Weiter vorteilhaft kann sein, dass ein außerhalb der Dekontaminationsvorrichtung angeordneter Sensor größer sein kann als ein Sensor innerhalb der Dekontaminationsvorrichtung.

Der Sensor kann beispielsweise auch ein Volumenstromsensor sein, welcher messen kann, wie viel H2O2 eingebracht wurde.

Bei einer vorteilhaften Ausgestaltung kann vorgesehen sein, dass der wenigstens eine Sensor einen Prüfsensor hat, mit dem die Dekontaminationsvorrichtung prüfbar ist. Somit kann beispielsweise die Funktion der Dekontaminationsvorrichtung geprüft werden. Insbesondere kann der wenigstens eine Sensor ein Prüfungsfeld haben, mit dem die Dekontaminationsvorrichtung prüfbar ist. Somit kann das Prüfungsfeld beispielsweise die Funktion der Dekontaminationsvorrichtung prüfen, indem die Dekontaminationsvorrichtung an das Prüfungsfeld gehalten wird. Das Prüfungsfeld kann zwischen mehreren Sensoren zur Überprüfung der Dekontaminationsvorrichtung aufgespannt sein. Alternativ oder zusätzlich kann vorgesehen sein, dass der wenigstens eine Sensor einen Indikations-Sensor hat, der eine Überprüfung auslöst. Hierdurch können beispielsweise mehrere Sensoren vorhanden sein, wobei ein Sensor einen Indikations-Sensor hat, der eine Überprüfung der Dekontaminationsvorrichtung auslösen kann. Vorteilhaft können somit Prozessparameter erfasst werden, die auf zuvor ermittelten validierten Parametern beruhen, wobei die Prozessparameter eine Grundlage für eine wiederkehrende Dekontamination sicherstellen.

Bei einer vorteilhaften Ausgestaltung kann vorgesehen sein, dass mit dem wenigstens einen Sensor wenigstens eine Stelle der Dekontaminationsvorrichtung, vorzugsweise mit potenziell geringster Dekontaminationswirkung, überprüfbar ist. Die Dekontaminationsvorrichtung kann beispielsweise nicht an jeder Stelle einheitlich gut dekontaminieren, sondern es können Stellen vorhanden sein, wo die Dekontaminationsvorrichtung weniger effektiv dekontaminieren kann. Daher kann es von Vorteil sein mit dem wenigstens einen Sensor diese Stelle der potenziell geringsten Dekontaminationswirkung zu messen, da sich so feststellen lässt, ob die Stelle der potenziell geringsten Dekontaminationswirkung noch über einem vorgegebenen Parameter für die Funktion der Dekontamination liegt.

Bei einer vorteilhaften Ausgestaltung kann vorgesehen sein, dass der wenigstens eine Sensor zur Erfassung einer Quantität eines von der Dekontaminationsvorrichtung emittierten Dekontaminationsmittels eingerichtet ist. Hierdurch kann der Sensor beispielsweise einen Messwert einer Messgröße messen, womit Rückschlüsse auf die Funktion der Dekontaminationsvorrichtung möglich sein können.

Bei einer vorteilhaften Ausgestaltung kann vorgesehen sein, dass ein Erfassungsbereich der Überprüfungseinrichtung eine gleich große Oberfläche hat wie die Passage. Hierdurch kann beispielsweise eine Dekontaminationsvorrichtung überprüft werden, die eine Oberfläche hat, die der Oberfläche der Passage entspricht. Es ist somit die Funktion über den gesamten Bereich der Passage überwachbar. Alternativ kann vorgesehen sein, dass ein Erfassungsbereich eine größere Oberfläche hat als die Passage. Hierdurch kann beispielsweise sichergestellt werden, dass die Überprüfungseinrichtung die Dekontaminationsvorrichtung vollständig abdecken kann, wobei auch ein zusätzlicher Randbereich der Dekontaminationsvorrichtung abgedeckt werden kann. Der Erfassungsbereich kann dabei von einzelnen Messpunkten aufgespannt werden. Die Oberfläche der Passage kann eine Ebene einer Öffnung sein, durch die die Objekte in die kontrollierte Umgebung schleusbar sind.

Bei einer vorteilhaften Ausgestaltung kann vorgesehen sein, dass ein Erfassungsbereich der Überprüfungseinrichtung größer ist als ein Abstrahlbereich der Dekontaminationsvorrichtung. Somit kann beispielsweise sichergestellt sein, dass die Überprüfungseinrichtung den gesamten Abstrahlbereich der Dekontaminationsvorrichtung erfassen kann. Insbesondere kann vorgesehen sein, dass sogenannte worst-case Positionen abgedeckt werden. Worst-case Positionen können, Positionen sein, an denen die Dekontaminationsvorrichtung eine geringere Dekontaminationsleistung erbringt, beispielsweise in Ecken der Dekontaminationsvorrichtung.

Bei einer vorteilhaften Ausgestaltung kann vorgesehen sein, dass die Überprüfungseinrichtung ein für die Dekontamination sensitives Überprüfungsmittel an der Verpackung aufweist. Hierdurch kann beispielsweise erreichbar sein, dass die Überprüfungseinrichtung Bestandteil der Verpackung ist, die die Passage von außen verschließt. Bei der Dekontamination wird die Verpackung dekontaminiert, wobei beispielsweise im Anschluss direkt an der Verpackung festgestellt werden kann, ob die Dekontaminationsvorrichtung vorgabengemäß funktioniert. Insbesondere kann das Überprüfungsmittel ein Verbrauchsmaterial sein. Somit kann das Überprüfungsmittel beispielsweise nach der Überprüfung der Dekontaminationsvorrichtung entsorgt werden. Eine Auswertung des Überprüfungsmittels kann beispielsweise optisch erfolgen.

Bei einer vorteilhaften Ausgestaltung kann vorgesehen sein, dass eine Überprüfungseinrichtung in der Dekontaminationsvorrichtung angeordnet ist. Hierdurch kann beispielsweise die Überprüfungseinrichtung einteilig mit der Dekontaminationsvorrichtung ausgebildet sein. Ebenfalls vorteilhaft kann sein, dass die Überprüfungseinrichtung bei jedem Dekontaminationsvorgang die Dekontaminationsvorrichtung überprüfen kann. Die Überprüfungseinrichtung kann in der Dekontaminationseinrichtung integriert sein.

Bei einer vorteilhaften Ausgestaltung kann vorgesehen sein, dass mehrere Sensoren einzelnen Dekontaminationselementen zugeordnet sind. Dekontaminationselemente können beispielsweise einzelne UVC-Lampen sein oder einzelne Dekontaminationsmittelverteilvorrichtungen. Die Sensoren können beispielsweise zwischen UVC-Lampen angeordnet sein.

Bei einer vorteilhaften Ausgestaltung kann vorgesehen sein, dass die Überprüfungseinrichtung und die Dekontaminationsvorrichtung zueinander beweglich sind. Hierdurch kann beispielsweise erreichbar sein, dass die Dekontaminationsvorrichtung an die Überprüfungseinrichtung, oder umgekehrt, herangeführt werden kann. Insbesondere kann vorgesehen sein, dass die Überprüfungseinrichtung und die Dekontaminationsvorrichtung zu einem Wechsel zwischen einer Überprüfungsposition und einer Arbeitsposition zueinander beweglich sind. Eine Überprüfungsposition kann die Position sein, in der die Dekontaminationsvorrichtung überprüft wird. Eine Arbeitsposition kann die Position sein, in der die Dekontaminationsvorrichtung die Passage dekontaminiert. Hierdurch können beispielsweise in der Überprüfungsposition die Überprüfungseinrichtung und die Dekontaminationsvorrichtung zueinander bewegt werden, wobei in einer Arbeitsposition die Dekontaminationsvorrichtung und die Überprüfungseinrichtung voneinander beabstandet sein können. Wenn die Überprüfungseinrichtung in die Dekontaminationseinrichtung integriert ist, können Überprüfungsposition und Arbeitsposition identisch sein. Alternativ oder zusätzlich kann vorgesehen sein, dass die Überprüfungseinrichtung und die Dekontaminationsvorrichtung derart zueinander beweglich sind, dass die Dekontaminationsvorrichtung in einen oder aus einem Erfassungsbereich der Überprüfungseinrichtung bringbar ist. Hierdurch kann beispielsweise erreichbar sein, dass die Dekontaminationsvorrichtung in den oder aus dem Erfassungsbereich der Überprüfungseinrichtung bewegt werden kann und dort überprüfbar ist. Vorzugsweise kann zumindest die Überprüfungseinrichtung fest installiert sein, so dass die Dekontaminationsvorrichtung sich aus der Arbeitsposition in die Überprüfungsposition zu der Überprüfungseinrichtung hin bewegen kann und von der Überprüfungsposition in die Arbeitsposition von der Überprüfungseinrichtung weg bewegen kann.

Bei einer vorteilhaften Ausgestaltung kann vorgesehen sein, dass die Dekontaminationsvorrichtung zumindest zeitweise in die kontrollierte Umgebung verfahrbar ist. Hierdurch kann beispielsweise die Dekontaminationsvorrichtung in eine Umgebung höherer Reinheit und/oder mit höherem Druckniveau bringbar sein. Alternativ oder zusätzlich kann vorgesehen sein, dass die Überprüfungseinrichtung in der kontrollierten Umgebung angeordnet ist. Hierdurch kann die Überprüfungseinrichtung beispielsweise in der kontrollierten Umgebung mit höherer Reinheit und/oder höherem Druckniveau angeordnet sein.

Bei einer vorteilhaften Ausgestaltung kann vorgesehen sein, dass die Einschleusevorrichtung eine Vergleichseinheit aufweist, die zum Vergleich einer von dem wenigstens einen Sensor gemessenen Quantität mit einem Parameter eingerichtet ist. Hierdurch kann beispielsweise erreichbar sein, dass ein Rückschluss auf die Funktion der Dekontaminationsvorrichtung möglich ist. Insbesondere kann der Parameter ein Wertebereich und/oder ein Sollwert und/oder ein Minimalwert und/oder ein Grenzwert sein. Die Funktion der Dekontaminationseinrichtung kann sich aus den genannten Parametern ergeben. Beispielsweise kann eine Messung der Eingangsleistung einer UVC-Lampe als Indikator für einen Betriebszustand herangezogen werden.

Bei einer vorteilhaften Ausgestaltung kann vorgesehen sein, dass die oder eine Vergleichseinheit zu einem Vergleich eines Messergebnisses wenigstens eines Sensors, der in die Dekontaminationsvorrichtung integriert ist, mit einem Messergebnis wenigstens eines Sensors, der außerhalb der Dekontaminationsvorrichtung angeordnet ist, eingerichtet ist. Hierdurch kann beispielsweise ein Vergleich der beiden an den beiden Sensoren gemessenen Messwerte erfolgen, um die Funktion der Dekontaminationsvorrichtung abzuleiten.

Zur Lösung der eingangs genannten Aufgabe sind außerdem erfindungsgemäß die Merkmale des auf ein Verfahren gerichteten Anspruchs vorgeschlagen. Insbesondere wird somit zur Lösung der eingangs genannten Aufgabe bei dem Verfahren vorgeschlagen, dass eine Funktion einer Dekontamination überprüft wird. Eine Funktionsüberprüfung der Dekontamination kann beispielsweise durch das Überprüfen einer Dekontaminationsvorrichtung, die die Dekontamination vornimmt, erfolgen. Hierdurch kann beispielsweise sichergestellt werden, dass die Dekontamination effektiv und sicher abläuft. Beispielsweise kann ein Ausfall oder ein Abfall einer Leistung der Dekontamination durch Prüfung der Dekontaminationsvorrichtung frühzeitig bemerkt werden. Das Verfahren zur Einschleusung kann insbesondere mit der zuvor beschriebenen Einschleusevorrichtung durchgeführt werden.

Bei einer bevorzugten Anwendung kann vorgesehen sein, dass die Dekontaminationsvorrichtung einem Prüfsensor präsentiert wird. Hierdurch kann beispielsweise der Prüfsensor die Dekontaminationsvorrichtung prüfen und einen Messwert einer Messgröße messen aus dem sich die Funktion der Dekontaminationsvorrichtung ergibt. Die Dekontaminationsvorrichtung kann dem Prüfsensor beispielsweise in Zeitintervallen präsentiert werden. Somit kann beispielsweise nach einem vorab definiertem Plan die Dekontaminationsvorrichtung geprüft werden und ist somit unabhängig von einer bedarfsgesteuerten Prüfung. Alternativ oder zusätzlich kann die Dekontaminationsvorrichtung dem Prüfsensor nach Erreichen eines vorbestimmten Kriteriums präsentiert werden. Hierdurch kann beispielsweise die Dekontaminationsvorrichtung geprüft werden, sobald ein Abfall oder eine Änderung der Funktion der Dekontaminationsvorrichtung gemessen wird. Wenn ein Minimalwert in einer bestimmten Zeit nicht erreicht wird, kann vorgesehen sein, dass die Passage nicht für nachfolgende Prozessschritte freigegeben wird, insbesondere nicht für eine Handlingseinheit freigegeben wird, sondern eine erneute Dekontamination durchgeführt wird.

Bei einer bevorzugten Anwendung kann vorgesehen sein, dass die Emissionseinheit nach einem Freigeben eines Zugangs einem externen Sensor zur Ermittlung einer Strahlendosis präsentiert wird. Hierdurch kann beispielsweise die Emissionseinheit der Dekontaminationsvorrichtung zur Überprüfung an den externen Sensor geführt werden, der dann eine Strahlendosis der Emissionseinheit messen kann. Somit kann diese Strahlendosis in Bezug auf die Dekontamination gemessen werden. Alternativ oder zusätzlich kann die Emissionseinheit vor einem Verschließen des Zugangs dem externen Sensor zur Ermittlung der Strahlendosis präsentiert werden. Hierdurch kann beispielsweise erreichbar sein, dass die Emissionseinheit vor der Dekontamination überprüft wird. Alternativ oder zusätzlich kann die Emissionseinheit zwischen zwei Dekontaminationszyklen der kontrollierten Umgebung dem externen Sensor zur Ermittlung der Strahlendosis präsentiert werden. Hierdurch kann beispielsweise eine Überprüfung der Dekontamination zwischen den beiden Dekontaminationszyklen erreichbar sein, was vorteilhaft den Betrieb in der kontrollierten Umgebung nicht unterbricht.

Bei einer bevorzugten Anwendung kann vorgesehen sein, dass eine von dem Prüfsensor gemessene Quantität mit einem Parameter abgeglichen wird. Hierdurch kann beispielsweise erreichbar sein, dass ein Rückschluss auf die Funktion der Dekontaminationsvorrichtung möglich ist. Alternativ oder zusätzlich kann eine von dem Indikations-Sensor gemessene Quantität mit einem Parameter abgeglichen werden. Hierdurch kann beispielsweise eine Überprüfung der Dekontaminationsvorrichtung ausgelöst werden. Alternativ oder zusätzlich kann vorgesehen sein, dass der Parameter ein Wertebereich und/oder ein Sollwert und/oder ein Minimalwert und/oder ein Grenzwert ist. Die Funktion der Dekontaminationseinrichtung kann sich aus den genannten Parameter ergeben.

Bei einer bevorzugten Anwendung kann vorgesehen sein, dass die Vergleichseinrichtung bei einer Abweichung der gemessenen Quantität von dem Parameter einen Alarm ausgibt. Der Alarm kann beispielsweise ein optischer und/oder akustischer Hinweis sein. Alternativ oder zusätzlich kann vorgesehen sein, dass die Abweichung in einem Speicher vermerkt wird. Hierdurch kann beispielsweise eine Log-Datei erstellt werden und abgespeichert werden wann und mit welchen Werten (Quantität) die Abweichung stattgefunden hat.

Bei einer bevorzugten Anwendung kann vorgesehen sein, dass die Verpackung mit einem Dekontaminationsmittel beaufschlagt wird und anschließend von einer Handlingseinrichtung geöffnet wird. Hierdurch kann die Dekontamination der Verpackung erreichbar sein. Ebenfalls kann die Verpackung so geöffnet werden um Objekte, die im Innern der Verpackung gehalten sind in die kontrollierte Umgebung zu entnehmen. Die Handlingseinrichtung kann beispielsweise ein Roboter sein, der mit einer Schneidvorrichtung, beispielsweise einem Messer, die Verpackung, insbesondere die Abdeckung der Verpackung, aufschneidet. Die Verpackung kann dabei beispielsweise außerhalb der kontrollierten Umgebung verbleiben.

Bei einer bevorzugten Anwendung kann vorgesehen sein, dass die Dekontaminationsvorrichtung während dem Öffnen der Verpackung zumindest zeitweise der Überprüfungseinrichtung präsentiert wird. Hierdurch kann beispielsweise die Funktion der Dekontaminationsvorrichtung zeitgleich zu dem Arbeitsschritt des Öffnens geprüft werden, was ein effizientes Arbeiten in der kontrollierten Umgebung ermöglicht. Außerdem kann somit beispielsweise mit hoher Wahrscheinlichkeit festgestellt werden, ob die zuvor ausgeführte Dekontamination der Dekontaminationsvorrichtung wirksam war. Alternativ oder zusätzlich kann vorgesehen sein, dass die Dekontaminationsvorrichtung während dem Entnehmen eines Inhalts der Verpackung zumindest zeitweise der Überprüfungseinrichtung präsentiert wird. Hierdurch kann beispielsweise ein effizientes Arbeiten in der kontrollierten Umgebung möglich sein. Vorzugsweise kann die Dekontaminationsvorrichtung mittels der Handlingsvorrichtung der Überprüfungseinrichtung präsentiert werden. Die Dekontaminationsvorrichtung kann also beispielsweise an einer Handlingsvorrichtung angeordnet sein oder von einer Handlingsvorrichtung gehalten oder gegriffen werden, um der Überprüfungseinrichtung präsentiert zu werden. Präsentieren kann hierbei bedeuten, dass die Dekontaminationsvorrichtung an die Überprüfungseinrichtung herangeführt und "gezeigt" oder in einen Erfassungsbereich der Sensoren der Überprüfungseinrichtung gebracht wird, damit die Überprüfung durchgeführt werden kann.

Bei einer bevorzugten Anwendung kann vorgesehen sein, dass ein Messergebnis eines, insbesondere des zuvor beschriebenen, wenigstens einen Sensors, der in der Dekontaminationsvorrichtung angeordnet ist mit einem Messergebnis wenigstens eines Sensors, der außerhalb der Dekontaminationsvorrichtung angeordnet ist, verglichen wird.

Hierdurch können beispielsweise die an beiden Sensoren, dem in der Dekontaminationsvorrichtung und dem außerhalb, gemessenen Messwerte verglichen werden, wobei bei einer Abweichung ein Hinweis auf eine Fehlfunktion der Dekontaminationsvorrichtung oder der Sensoren vorliegen kann. Dies kann die Sicherheit der Dekontaminationsvorrichtung und damit eines Einschleuseprozesses erhöhen.

Bei einer bevorzugten Ausgestaltung kann vorgesehen sein, dass die Passage von außen mit einer Überprüfungseinrichtung verschlossen wird, wobei die Überprüfungseinrichtung Sensoren umfasst. Die Überprüfungseinrichtung kann dabei eine Attrappe einer Verpackung darstellen. Hierdurch kann vorteilhaft die Dekontaminationsvorrichtung in einer Prüfsituation überprüft werden, die der Arbeitsposition entspricht.

Die Erfindung wird nun anhand mehrerer Ausführungsbeispiele näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

Es zeigen:
- Fig. 1: eine Einschleusevorrichtung in einer seitlichen Schnittansicht in einer Arbeitsposition,
- Fig. 2: die Einschleusevorrichtung aus Fig. 1 in einer seitlichen Schnittansicht in einer Arbeitsposition,
- Fig. 3: die Einschleusevorrichtung aus Fig. 1 - 2 in einer seitlichen Schnittansicht in einer Überprüfungsposition,
- Fig. 4: die Einschleusevorrichtung aus Fig. 1 - 3 in einer Draufsicht in einer Arbeitsposition,
- Fig. 5: die Einschleusevorrichtung aus Fig. 1 - 4 in einer Draufsicht in einer Überprüfungsposition,
- Fig. 6: die Einschleusevorrichtung aus Fig. 1 - 5 in einer seitlichen Schnittansicht, wobei Sensoren in einer Dekontaminationsvorrichtung angeordnet sind,
- Fig. 7: eine Einschleusevorrichtung in einer seitlichen Schnittansicht, wobei die Dekontaminationsvorrichtung in einer Passage angeordnet ist,
- Fig. 8: eine Detailansicht der Passage aus Fig. 2,
- Fig. 9: eine Einschleusevorrichtung in einer seitlichen Schnittdarstellung mit einer Überprüfungs-Verpackung,
- Fig. 10: eine Verpackung mit Behältern im Innern,
- Fig. 11: eine weitere Verpackung mit Behältern im Innern,
- Fig. 12: die Verpackung aus Fig. 11 in einem geöffneten Zustand
- Fig. 13: eine Ansicht auf eine Deckelunterseite und eine UVC-Lampe.

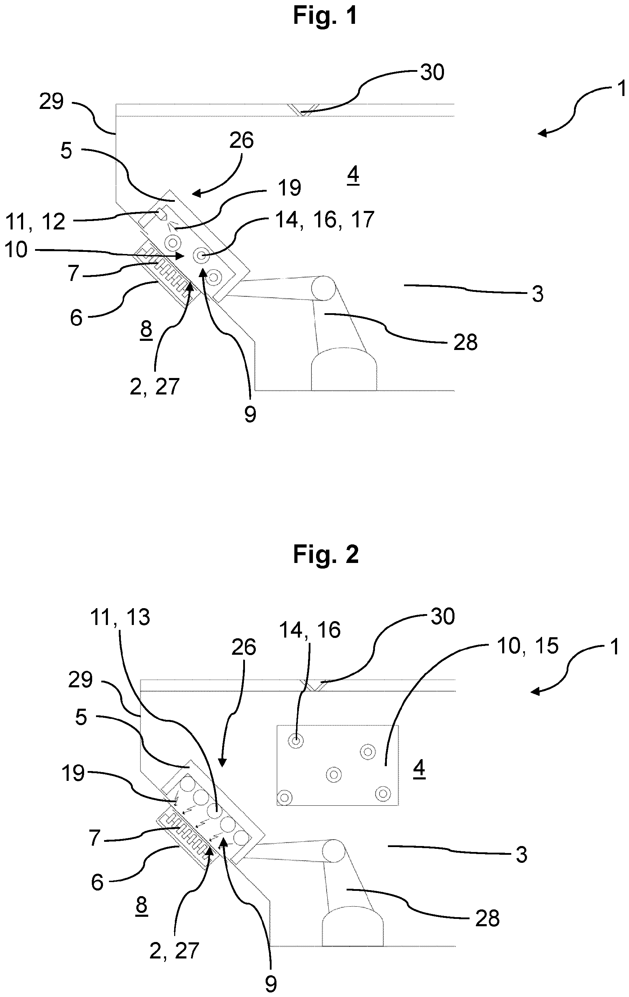

Fig. 1 zeigt die im Gesamten mit 1 bezeichnete Einschleusevorrichtung 1. Die Einschleusevorrichtung 1 umfasst eine kontrollierte Umgebung 3 mit einer Passage 2. Die Passage 2 ist von innen 4 mit einem Deckel 5 verschlossen. Die Passage 2 ist mit einer Verpackung 6, die ein einzuschleusendes Objekt 7 enthält, von außen 8 verschlossen. Die Einschleusevorrichtung 1 umfasst eine Dekontaminationsvorrichtung 9, die zur Dekontamination der Passage 2, sowie der die Passage abschließenden Oberflächen, eingerichtet ist. Die in den Fig. 1 - 7 und 9 gezeigte kontrollierte Umgebung ist ein Isolator 29 der zur rechten Bildseite hin offen gezeichnet ist. Die Darstellungen dienen der Illustration, wobei der Isolator 29 selbstverständlich nicht seitlich offen ist. Insofern ist auch klar, dass der Innenbereich 4 des Isolators 29 vom Außenbereich 8 getrennt ist.

Fig. 2 zeigt die Einschleusevorrichtung 1, die zur Überprüfung einer Funktion der Dekontaminationsvorrichtung 9 eine Überprüfungsvorrichtung 10 umfasst. Die Dekontaminationsvorrichtung 9 umfasst eine Emissionseinheit 11, die im Ausführungsbeispiel der Fig. 1 als H2O2 Vernebler 12 und im Ausführungsbeispiel der Fig. 2 als UVC-Lampe 13 ausgebildet ist. Der H2O2 Vernebler 12 kann H2O2 in der Passage 2 verteilen und die Passage 2 somit dekontaminieren. Die UVC-Lampe 13 kann mittels UVC-Strahlung die Passage 2 dekontaminieren. Bei beiden Dekontaminationsverfahren können mikrobiologische Verunreinigungen entfernt und/oder abgetötet werden.

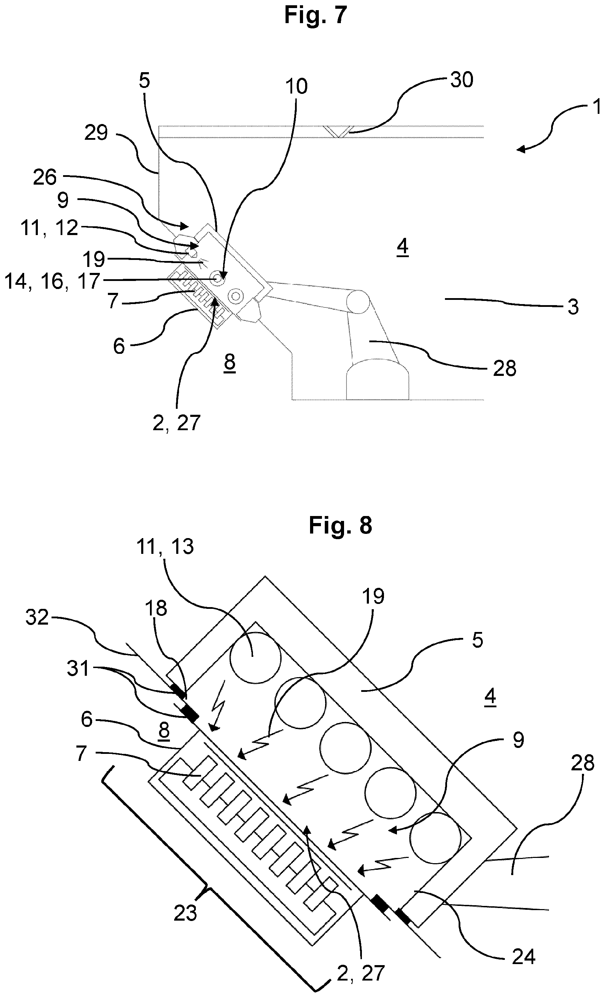

In den in den Fig. 1, 2, 3, 6 und 9 vorliegenden Ausführungsbeispielen ist die Dekontaminationsvorrichtung 9 in dem Deckel 5 gezeigt. In dem in Fig. 7 gezeigten Ausführungsbeispiel ist die Dekontaminationsvorrichtung 9 in der Passage 2 angeordnet.

In den in Fig. 1, 2, 3, 4, 5, 6 und 9 gezeigten Ausführungsbeispielen ist die Überprüfungseinrichtung 10 außerhalb der Passage 2 angeordnet. In dem in Fig. 7 gezeigten Ausführungsbeispiel ist die Überprüfungseinrichtung 10 innerhalb der Passage 2 angeordnet.

Die Überprüfungseinrichtung 10 weist mehrere Sensoren 14 auf, wobei Sensoren 14 in Fig. 1 in der Dekontaminationsvorrichtung 9 angeordnet sind. In der in Fig. 2, 3, 4 und 5 gezeigten Einschleusevorrichtung 1 sind die Sensoren 14 außerhalb der Dekontaminationsvorrichtung 9 angeordnet. Dort sind die Sensoren 14 in der kontrollierten Umgebung 3 angeordnet, wobei die Sensoren 14 in einem Prüfungsfeld 15 angeordnete Prüfsensoren 16 umfassen mit dem die Dekontaminationsvorrichtung 9 prüfbar ist.

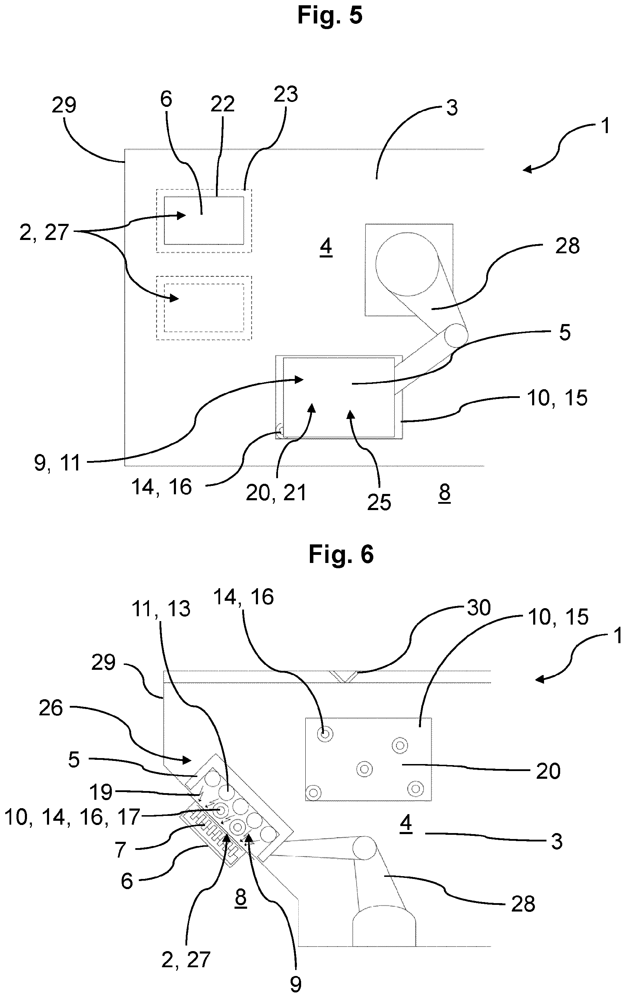

Die Sensoren 14 umfassen ebenfalls Indikationssensoren 17 die eine Überprüfung der Dekontaminationsvorrichtung 9 auslösen können. Die Indikationssensoren 17 sind in dem Ausführungsbeispiel der Fig. 6 in die Dekontaminationsvorrichtung 9 integriert die in dem Deckel 5 angeordnet ist.

Die Sensoren 14 können eine Stelle 18 der Dekontaminationsvorrichtung 9 mit potenziell geringster Dekontaminationswirkung überprüfen.

Die Sensoren 14 sind zur Erfassung einer Quantität eines von der Dekontaminationsvorrichtung 9 emittierten Dekontaminationsmittels 19 eingerichtet.

Die Überprüfungseinrichtung 10 hat einen Erfassungsbereich 20 der eine größere Oberfläche 21 als eine Oberfläche 22 der Passage 2 hat.

Der Erfassungsbereich 20 der Überprüfungseinrichtung 10 ist größer als ein Abstrahlbereich 23 der Dekontaminationsvorrichtung 9. Somit kann die Überprüfungseinrichtung 10 sogenannte worst-case Positionen 24 der Dekontaminationsvorrichtung 9 erfassen.

In einem nicht gezeigten Ausführungsbeispiel weist die Verpackung 6 ein für die Dekontamination sensitives Überprüfungsmittel auf. Dies kann beispielsweise ein Label oder eine Bedruckung sein, das bzw. die sich unter einer Einwirkung des Dekontaminationsmittels optisch erfassbar verändert.

Die Überprüfungseinrichtung 10 ist in dem in Fig. 1 und 6 gezeigten Ausführungsbeispiel in der Dekontaminationsvorrichtung 9 angeordnet.

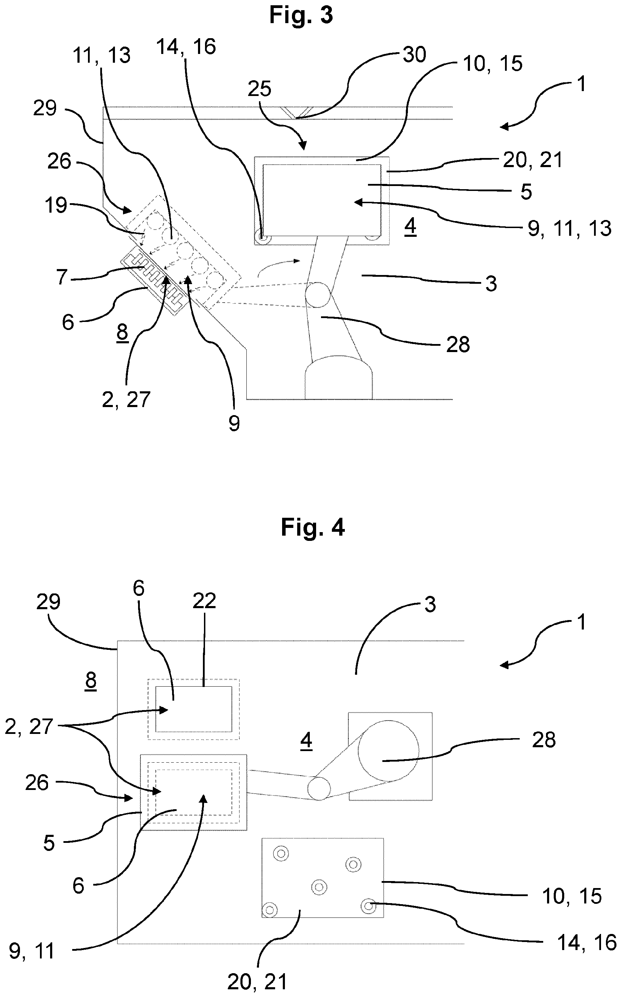

Fig. 3 zeigt, wie die Überprüfungseinrichtung 10 und die Dekontaminationsvorrichtung 9 zueinander beweglich sind, um von einer Überprüfungsposition 25 in eine Arbeitsposition 26, und umgekehrt, wechseln zu können. Die

Dekontaminationsvorrichtung 9 ist so in einen oder aus einem Erfassungsbereich 20 der Überprüfungseinrichtung 10 bringbar. In Fig. 3 bewegt sich die Dekontaminationsvorrichtung 9 von der Arbeitsposition 26 in die Überprüfungsposition 25.

Die Dekontaminationsvorrichtung 9 ist zeitweise in die kontrollierte Umgebung 3 verfahrbar. Die Überprüfungseinrichtung 10 ist in der kontrollierten Umgebung 3 angeordnet.

In einem nicht gezeigten Ausführungsbeispiel weist die Einschleusevorrichtung 1 eine Vergleichseinheit auf, die zum Vergleich einer mit einem Sensor 15 gemessenen Quantität mit einem Parameter eingerichtet ist. Der Parameter kann ein Wertebereich und/oder Sollwert und/oder Minimalwert und/oder Grenzwert sein.

Die Vergleichseinheit ist zu einem Vergleich eines Messergebnisses der Sensoren 14 die in der Dekontaminationsvorrichtung 9 integriert sind und eines Messergebnisses von einem Sensor 14 außerhalb der Dekontaminationsvorrichtung 9 eingerichtet.

Zum Einschleusen eines Objekts 7 in die kontrollierte Umgebung 3 wird die Verpackung 6 des Objekts 7 einer Dekontamination ausgesetzt, bevor das Objekt 7 in die kontrollierte Umgebung 3 gebracht wird, wobei eine Funktion der Dekontamination überprüft wird. Die Funktion meint dabei ein vorgabengemäßes Funktionieren der Dekontaminationsvorrichtung 9, so dass die Verpackung 6 dekontaminiert werden kann. Die Verpackung 6 des Objekts 7 wird mit der Dekontaminationsvorrichtung 9 dekontaminiert, bevor das Objekt 7 in die kontrollierte Umgebung 3 geschleust wird. Vor, während oder nach dem Einschleusen kann dabei eine Funktionsprüfung der Dekontaminationsvorrichtung 9 mit der Überprüfungsvorrichtung 10 erfolgen. Die Dekontaminationsvorrichtung 9 wird dem Prüfsensor 16 präsentiert. Dies kann in Zeitintervallen und/oder nach Erreichen eines vorbestimmten Kriteriums geschehen. Beispielsweise kann ein vorbestimmtes Kriterium ein Messwert des Indikationssensors 17 sein, wodurch die Überprüfung ausgelöst wird.

Die Emissionseinheit 11 wird nach einem Freigeben eines Zugangs 27 und/oder vor einem Verschließen des Zugangs 27 und/oder zwischen zwei Dekontaminationszyklen der kontrollierten Umgebung 3 oder nach einem durch den Anlagenbetreiber definierten Zeitraum einem externen Sensor 14 zur Ermittlung einer Strahlendosis präsentiert.

Eine von dem Prüfsensor 16 und/oder Indikationssensor 17 gemessene Quantität wird mit einem Parameter abgeglichen. Der Parameter ist ein Wertebereich und/oder ein Sollwert und/oder ein Minimalwert und/oder ein Grenzwert.

Die Vergleichseinheit gibt bei einer Abweichung der gemessenen Quantität von dem Parameter einen Alarm aus. Ebenfalls vermerkt die Vergleichseinheit die Abweichung in einem Speicher und/oder Protokoll. Es findet ein Vergleich zwischen einem Messergebnis das von dem Sensor 14, der in der Dekontaminationsvorrichtung 9 angeordnet ist, gemessen wird und einem Messergebnis das von einem Sensor 14, der außerhalb der Dekontaminationsvorrichtung 9 angeordnet ist, gemessen wird, statt.

Zur Dekontamination der Verpackung 6 wird die Verpackung 6, oder zumindest Teile davon, mit einem Dekontaminationsmittel 19 beaufschlagt und anschließend von einer Handlingseinrichtung geöffnet. Ein Beaufschlagen kann das Ausbringen des Dekontaminationsmittels sein.

Die Dekontaminationsvorrichtung 9 kann, wenn nötig, während dem Öffnen der Verpackung 6 und/oder dem Entnehmen eines Inhalts der Verpackung 6 mittels der Handlingsvorrichtung 28 zeitweise der Überprüfungseinrichtung 10 präsentiert werden. Die Dekontaminationsvorrichtung 9 ist an der Handlingsvorrichtung 28 angeordnet und kann mit dieser bewegt werden.

Die kontrollierte Umgebung 3 ist im vorliegenden Ausführungsbeispiel ein Isolator 29. Die kontrollierte Umgebung 3 weist eine laminare Luftströmung 30 auf, die vorzugsweise entlang einer Richtung der Gravitationskraft, und/oder Ausblasrichtung entspricht, fließt.

Fig. 4 zeigt eine kontrollierte Umgebung 3 mit zwei Passagen 2, wobei eine erste Passage 2 gerade von der Dekontaminationsvorrichtung 9 dekontaminiert wird. Die zweite Passage 2 ist von der Verpackung 6 von außen 8 verschlossen. Während die erste Passage 2 mit einer daran von außen 8 angepressten Verpackung 6 dekontaminiert wird, kann die Verpackung 6 die in der zweiten Passage 2 gehalten ist, von einer nicht gezeigten Handlingseinrichtung geöffnet, beispielsweise aufgeschnitten oder gestanzt werden.

Fig. 5 zeigt die Überprüfungsposition 25, wobei die an der Handlingsvorrichtung 28 gelagerte Dekontaminationsvorrichtung 9 an die Überprüfungsvorrichtung 10 bewegt ist und dort von den Sensoren 14, die Prüfsensoren 16 sind, des Prüfungsfelds 15 geprüft wird.

Die in Fig. 6 gezeigte Dekontaminationsvorrichtung 9 umfasst neben der Emissionseinheit 11 auch Sensoren 14, die als Indikationssensoren 17 und Prüfsensoren 16 ausgebildet sind.

In einem nicht gezeigten Ausführungsbeispiel sind die Sensoren 14 der Emissionseinheit 11 als Indikationssensoren 17 ausgestaltet. In einem weiteren nicht gezeigten Ausführungsbeispiel sind die Sensoren 14 der Emissionseinheit 11 als Prüfsensoren 15 ausgebildet.

Fig. 8 zeigt eine Detailansicht der Passage 2 der Einschleusevorrichtung 1 aus Fig. 2. In der Detailansicht sind Dichtungen 31 zwischen der Verpackung 6 und einer Gehäusewand 32 der kontrollierten Umgebung 3 und zwischen dem Deckel 5 und der Gehäusewand 32 skizziert, die verdeutlichen, dass kein Luftaustausch zwischen außen 8 und innen 4 der kontrollierten Umgebung 3 stattfinden kann. In einer nicht gezeigten Form ist eine einstückige Dichtung, vorzugsweise in zulaufender Form zur Verhinderung eines Schattenwurfs angeordnet. In den anderen Figuren sind die Dichtungen 31 ebenfalls vorhanden, aber nicht gezeigt. Fig. 8 zeigt auch beispielhaft eine Stelle 18, wo die Dekontaminationsvorrichtung 9 potenziell eine geringste Dekontaminationswirkung aufweist. Beispielhaft ist in der Dekontaminationsvorrichtung 9 eine worst-case Position 24 am Rand der Dekontaminationsvorrichtung 24 gezeigt. Hier kann die Dekontaminationsvorrichtung 9 eine geringere Dekontaminationsleistung erbringen als im Zentrum der Dekontaminationsvorrichtung 9. Der Abstrahlbereich 23 ist kleiner als der Erfassungsbereich 20 der Überprüfungsvorrichtung 10 (siehe Fig. 5).

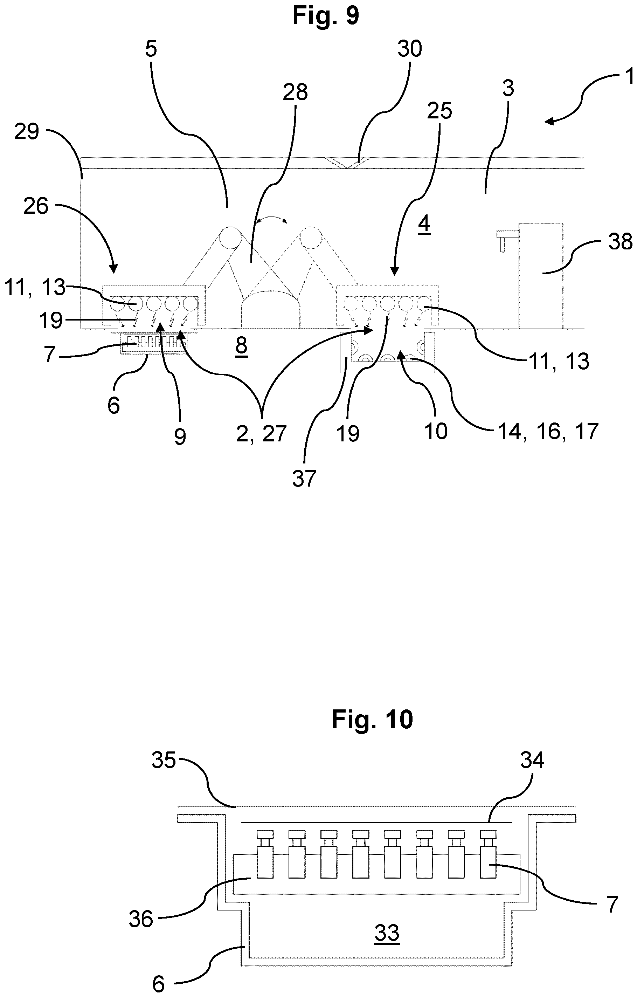

Fig. 9 zeigt eine Einschleusevorrichtung 1 einer kontrollierten Umgebung 3. Zur Überprüfung der Funktion der Dekontaminationsvorrichtung 9 ist eine Überprüfungseinrichtung 10 in Form einer Überprüfungs-Verpackung 37, von außen 8 an die Passage 2 gehalten. In einer Überprüfungsposition 25 bewegt die Handlingsvorrichtung 28 die Dekontaminationsvorrichtung 9 an die Passage 2. Bei der Überprüfung der Dekontaminationsvorrichtung 9 arbeitet die Emissionseinheit 11 so wie sie in der Arbeitsposition 26 mit einer Verpackung 6 arbeiten würde. In der Überprüfungs-Verpackung 37 sind Sensoren 14 angeordnet, wobei die Sensoren 14 Prüfsensoren sind. Mit Hilfe der Überprüfungs-Verpackung 37 lässt sich vorteilhaft die Funktion der

Dekontaminationsvorrichtung 9 an der Verpackung 6 simulieren. In der gezeigten kontrollierten Umgebung 3 ist eine Prozessstation in Form einer Befüllvorrichtung 38 angeordnet.

Fig. 10 zeigt die Verpackung 6 in einer seitlichen Schnittansicht, wobei die Verpackung 6 eine Behälteraufnahme 36 mit Objekten 7 enthält. Die Objekte 7 sind Behälter für die Abfüllung oder Weiterverarbeitung, beispielsweise von medizinischen Produkten. Die Objekte 7 sind von einer Innen-Abdeckung 34 abgedeckt. Die Verpackung 6 umfasst eine Abdeckung 35, wobei diese Abdeckung 35 der Dekontamination ausgesetzt wird. Ein Innenraum 33 der Verpackung 6 ist aseptisch. Die Abdeckung 35 ist dichtend mit der Verpackung 6 verbunden.

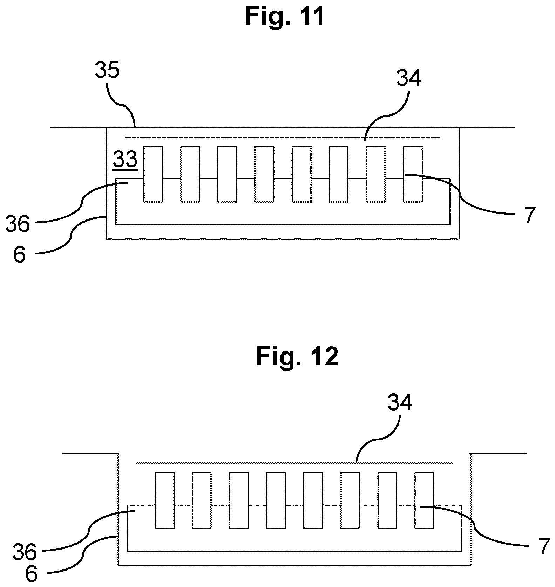

Fig. 11 zeigt eine weitere Ausgestaltung der Verpackung 6 in einer seitlichen Schnittansicht, wobei die Verpackung 6 eine Behälteraufnahme 36 mit Objekten 7 enthält.

Fig. 12 zeigt die Verpackung 6 ohne die Abdeckung 35.

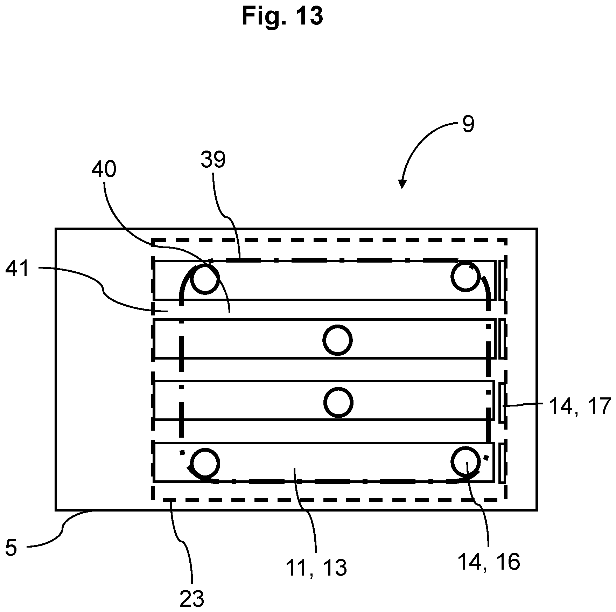

Fig. 13 zeigt eine Ansicht auf eine Unterseite des Deckels 5, wobei die Dekontaminationsvorrichtung 9 in dem Deckel 5 angeordnet ist und Leuchtröhren einer UVC-Lampe 13 umfasst. Die Dekontaminationsvorrichtung 9 umfasst auch Sensoren 14, die als Prüfsensoren 16 und Indikationssensoren 17 ausgestaltet sind. Die gestrichpunktete Linie entspricht einem Umfang 39 der Passage 2 auf die der Deckel 5 zur Dekontamination aufgesetzt wird. Die Prüfsensoren 16 erfassen eine Lichtstärke der UVC-Lampe 13, wobei jeweils ein Prüfsensor 16 in einer Ecke einer Fläche 40 angeordnet ist, die durch die gestrichpunktete Linie umgrenzt wird. Zwei weitere Prüfsensoren 16 sind im Zentrum der Fläche angeordnet.

Die in Fig. 13 gestrichelt dargestellte Linie entspricht dem Abstrahlbereich 23 der Dekontaminationsvorrichtung 9. Eine Fläche 41 des Abstrahlbereichs 23 ist größer als die Fläche 40 der Passage 2. Die Dekontaminationsvorrichtung 9 weist Indikationssensoren 17 auf, die neben der UVC-Lampe 13 angeordnet sind.

Es wird ein Verfahren zum Einschleusen von Objekten 7 in eine kontrollierte Umgebung 3 und eine Einschleusevorrichtung 1 mit einer eine Passage 2 aufweisenden kontrollierten Umgebung 3 vorgeschlagen, wobei die Passage 2 mit einem Deckel 5 von innen 4, insbesondere innerhalb der kontrollierten Umgebung 3, und mit einer ein einzuschleusendes Objekt 7 enthaltenden Verpackung 6 von außen 8 verschließbar ist, und mit einer Dekontaminationsvorrichtung 9, die zu einer Dekontamination der Passage 2 eingerichtet ist, wobei eine Überprüfungseinrichtung 10 zu einer Überprüfung einer Funktion der Dekontaminationsvorrichtung 9 eingerichtet ist.

### Bezugszeichenliste

- 1: Einschleusevorrichtung
- 2: Passage
- 3: Kontrollierte Umgebung
- 4: Innen
- 5: Deckel
- 6: Verpackung
- 7: Objekt
- 8: Außen
- 9: Dekontaminationsvorrichtung
- 10: Überprüfungsvorrichtung
- 11: Emissionseinheit
- 12: H2O2 Vernebler
- 13: UVC-Lampe
- 14: Sensor
- 15: Prüfungsfeld
- 16: Prüfsensor
- 17: Indikationssensor
- 18: Stelle
- 19: Dekontaminationsmittel
- 20: Erfassungsbereich der Überprüfungsvorrichtung
- 21: Oberfläche des Erfassungsbereichs
- 22: Oberfläche der Passage
- 23: Abstrahlbereich
- 24: Worst-case Position
- 25: Überprüfungsposition
- 26: Arbeitsposition
- 27: Zugang
- 28: Handlingsvorrichtung
- 29: Isolator
- 30: Luftströmung
- 31: Dichtung
- 32: Gehäusewandung
- 33: Innenraum der Verpackung
- 34: Innen-Abdeckung
- 35: Abdeckung
- 36: Behälteraufnahme
- 37: Überprüfungs-Verpackung
- 38: Befüllvorrichtung
- 39: Umfang
- 40: Fläche der Passage
- 41: Fläche des Abstrahlbereichs

## Patentansprüche

1. Einschleusevorrichtung (1) mit einer eine Passage (2) aufweisenden kontrollierten Umgebung (3), wobei die Passage (2) mit einem Deckel (5) von innen (4), insbesondere innerhalb der kontrollierten Umgebung (3), und mit einer ein einzuschleusendes Objekt (7) enthaltenden Verpackung (6) von außen (8) verschließbar ist, und mit einer Dekontaminationsvorrichtung (9), die zu einer Dekontamination der Passage (2) eingerichtet ist, **dadurch gekennzeichnet, dass** eine Überprüfungseinrichtung (10) zu einer Überprüfung zumindest einer Funktion der Dekontaminationsvorrichtung (9) eingerichtet ist.

2. Einschleusevorrichtung (1) nach Anspruch 1, **dadurch gekennzeichnet, dass** die Dekontaminationsvorrichtung (9) eine Emissionseinheit (11), insbesondere einen Strahlungsgenerator, vorzugsweise eine UVC-Lampe (13), und/oder eine Dekontaminationsmittelverteilvorrichtung, insbesondere einen H2O2-Vernebler (12), aufweist.

3. Einschleusevorrichtung (1) nach einem der vorangehenden Ansprüche, **dadurch gekennzeichnet, dass** die Dekontaminationsvorrichtung (9) in dem Deckel (5) und/oder in der Passage (2) angeordnet ist.

4. Einschleusevorrichtung (1) nach einem der vorangehenden Ansprüche, **dadurch gekennzeichnet, dass** die Überprüfungseinrichtung (10) innerhalb der Passage (2) und/oder außerhalb der Passage (2) angeordnet ist.

5. Einschleusevorrichtung (1) nach einem der vorangehenden Ansprüche, **dadurch gekennzeichnet, dass** die Überprüfungseinrichtung (10) wenigstens einen Sensor (14) aufweist, insbesondere wobei der wenigstens eine Sensor (14) in der Dekontaminationsvorrichtung (9) angeordnet ist und/oder wobei der wenigstens eine Sensor (14) außerhalb der Dekontaminationsvorrichtung (9) angeordnet ist.

6. Einschleusevorrichtung (1) nach einem der vorangehenden Ansprüche, **dadurch gekennzeichnet, dass** der wenigstens eine oder wenigstens ein Sensor (14) einen Prüfsensor (16), insbesondere ein Prüfungsfeld (15), hat, mit dem die Dekontaminationsvorrichtung (9) prüfbar ist, und/oder dass der wenigstens eine Sensor (14) einen Indikationssensor (17) hat, der eine Überprüfung auslöst.

7. Einschleusevorrichtung (1) nach einem der vorangehenden Ansprüche, **dadurch gekennzeichnet, dass** die Überprüfungseinrichtung (10) ein für die Dekontamination sensitives Überprüfungsmittel an der Verpackung (6) aufweist, insbesondere als Verbrauchsmaterial.

8. Einschleusevorrichtung (1) nach einem der vorangehenden Ansprüche, **dadurch gekennzeichnet, dass** die Überprüfungseinrichtung (10) in der Dekontaminationsvorrichtung (9) angeordnet ist.

9. Einschleusevorrichtung (1) nach einem der vorangehenden Ansprüche, **dadurch gekennzeichnet, dass** die Überprüfungseinrichtung (10) und die Dekontaminationsvorrichtung (9) zueinander beweglich sind, insbesondere zu einem Wechsel zwischen einer Überprüfungsposition (25) und einer Arbeitsposition (26) und/oder derart, dass die Dekontaminationsvorrichtung (9) in einen oder aus einem Erfassungsbereich (20) der Überprüfungseinrichtung (10) bringbar ist.

10. Einschleusevorrichtung (1) nach einem der vorangehenden Ansprüche, **dadurch gekennzeichnet, dass** die Dekontaminationsvorrichtung (9) zumindest zeitweise in die kontrollierte Umgebung (3) verfahrbar ist und/oder dass die Überprüfungseinrichtung (10) in der kontrollierten Umgebung (3) angeordnet ist.

11. Einschleusevorrichtung (1) nach einem der vorangehenden Ansprüche, **dadurch gekennzeichnet, dass** die Einschleusevorrichtung (1) eine Vergleichseinheit aufweist, die zum Vergleich einer von wenigstens einem Sensor (14) gemessenen Quantität mit einem Parameter, insbesondere einem Wertebereich und/oder einem Sollwert und/oder einem Minimalwert und/oder einem Grenzwert, eingerichtet ist.

12. Verfahren zum Einschleusen eines Objektes (7) in eine kontrollierte Umgebung (3), insbesondere mit der Einschleusevorrichtung (1) nach einem der vorangehenden Ansprüche, wobei das Objekt (7) in einer Verpackung (6) gehalten ist, die eine Passage (2) von außen verschließt, wobei zumindest ein Teil der Verpackung (6) einer Dekontamination ausgesetzt wird, bevor das Objekt (7) in die kontrollierte Umgebung (3) gebracht wird, **dadurch gekennzeichnet, dass** eine Funktion der Dekontamination überprüft wird.

13. Verfahren nach einem der vorangehenden Ansprüche, **dadurch gekennzeichnet, dass** die Dekontaminationsvorrichtung (9) einem Prüfsensor (16), beispielsweise in Zeitintervallen und/oder nach Erreichen eines vorbestimmten Kriteriums, präsentiert wird.

14. Verfahren nach einem der vorangehenden Ansprüche, **dadurch gekennzeichnet, dass** die Emissionseinheit (11) nach einem Freigeben eines Zugangs (27) und/oder vor einem Verschließen des Zugangs (27) und/oder zwischen zwei Dekontaminationszyklen der kontrollierten Umgebung (3) einem externen Sensor (28) zur Ermittlung einer Strahlendosis präsentiert wird.

15. Verfahren nach einem der vorangehenden Ansprüche, **dadurch gekennzeichnet, dass** eine von dem Prüfsensor (16) und/oder Indikationssensor (17) gemessene Quantität mit einem Parameter, insbesondere einem Wertebereich und/oder einem Sollwert und/oder einem Minimalwert und/oder einem Grenzwert abgeglichen wird.

16. Verfahren nach einem der vorangehenden Ansprüche, **dadurch gekennzeichnet, dass** die Vergleichseinheit bei einer Abweichung der gemessenen Quantität von dem Parameter einen Alarm ausgibt und/oder die Abweichung in einem Speicher vermerkt.

17. Verfahren nach einem der vorangehenden Ansprüche, **dadurch gekennzeichnet, dass** die Dekontaminationsvorrichtung (9) während dem Öffnen der Verpackung (6) und/oder dem Entnehmen eines Inhalts der Verpackung (6), vorzugsweise mittels der Handlingsvorrichtung (28), zumindest zeitweise der Überprüfungseinrichtung (10) präsentiert wird.

18. Verfahren nach einem der vorangehenden Ansprüche, **dadurch gekennzeichnet, dass** ein Messergebnis des oder eines wenigstens einen Sensors (14), der in der Dekontaminationsvorrichtung (9) angeordnet ist mit einem Messergebnis wenigstens eines Sensors (14), der außerhalb der Dekontaminationsvorrichtung (9) angeordnet ist, verglichen wird.
